Europäisches Patentamt

(19) European Patent Office

Office européen des brevets

(11) Numéro de publication: **0 228 975**
**A1**

(12) ## DEMANDE DE BREVET EUROPEEN

(21) Numéro de dépôt: 86420292.4

(22) Date de dépôt: 26.11.86

(51) Int. Cl.⁴: **C 07 K 15/00**
C 12 P 21/00, A 61 K 39/42
G 01 N 33/02, G 01 N 33/04
G 01 N 33/569
//C12N15/00, (C12P21/00,
C12R1:19)

(30) Priorité: 27.11.85 FR 8518207

(43) Date de publication de la demande:
15.07.87 Bulletin 87/29

(84) Etats contractants désignés:
AT BE CH DE ES FR GB IT LI LU NL SE

(71) Demandeur: TRANSIA, Société anonyme dite
119 avenue de Saxe
F-69003 Lyon(FR)

(72) Inventeur: Bonneau, Marc
32 rue des Granges
Lyon (5ème) (Rhône)(FR)

(74) Mandataire: Maureau, Philippe et al,
Cabinet Germain & Maureau Le Britannia - Tour C 20, bld
Eugène Déruelle
F-69003 Lyon(FR)

(54) Mélange d'anticorps spécifiques des bactériophages de bactéries lactiques et ses applications à la détection et à la neutralisation desdits bactériophages.

(57) Ce mélange d'anticorps anti-bactériophages lactiques est obtenu par immunisation d'animaux à l'aide d'antigènes constitués par des sérotypes de bactériophages lactiques rencontrés dans la nature ou en laboratoire et actifs contre les bactéries lactiques mésophiles et thermophiles telles que les Streptocoques lactiques, les Lactobacilles, les Pédiocoques et les Leuconostocs.

L'invention concerne également ses applications à titre curatif et neutralisant dans les milieux biologiques tels que les laits ou les cultures bactériennes initialement contaminées par les bactériophages, ainsi que ses applications à la détection immunologique des bactériophages présents dans un milieu contaminé (levain, lait, lacto-sérum, etc...).

EP 0 228 975 A1

1

# MELANGE D'ANTICORPS SPECIFIQUES DES BACTERIOPHAGES
# DE BACTERIES LACTIQUES ET SES APPLICATIONS
# A LA DETECTION ET A LA NEUTRALISATION
# DESDITS BACTERIOPHAGES

La présente invention concerne un mélange d'anticorps spécifiques des bactériophages de bactéries lactiques et les applications de ce mélange à la détection et à la neutralisation desdits bactériophages.

Les aliments fermentés, tels que le pain et le vin, constituent depuis des siècles, une des bases essentielles de l'alimentation humaine. L'obtention des aliments fermentés fait intervenir des micro-organismes tels que les levures et les bactéries qui utilisent et transforment les substrats carbonés des matières premières en métabolites divers comme l'alcool et l'acide lactique ; c'est ainsi que les bactéries lactiques, telles que Streptocoques lactiques, Lactobacilles, Pédiocoques et Leuconostocs, responsables de la fermentation lactique, transforment, par fermentation, les sucres tels que le lactose en acide lactique, et confèrent aux aliments fermentés des qualités organo-leptiques et alimentaires fortement améliorées et souvent nouvelles.

Les bactéries lactiques interviennent ainsi dans la fabrication de nombreux produits alimentaires tels que les salaisons et les conserves, les marinades, les choucroutes, les fromages, les vins ainsi que dans l'obtention de divers produits fermentés particuliers à certains pays.

Le succès des fabrications industrielles de ces aliments dépend donc étroitement de la bonne conduite du processus de fermentation lactique lui-même lié à une vitalité suffisante des souches bactériennes utilisées.

Il arrive cependant que ces souches bactériennes (genre Strepto-ccocus, genre Lactobacillus, genre Pédiococcus, genre Leuconostoc) soient attaquées et détruites par des virus bactériophages, ce qui aboutit à un accident ou à une absence de fermentation et qui entraîne, par là même, des irrégularités de fabrication et des déclassements de production aux conséquences économiques importantes.

Dans l'industrie laitière, divers procédés de lutte contre les attaques phagiques des bactéries lactiques ont été proposés avec des succès divers et irréguliers (milieux de culture carencés en tryptophane, utilisation de mélanges de souches bactériennes à sensibilité variable vis-à-vis des phages, etc...).

2      0228975

A l'heure actuelle, les accidents de fabrications fromagères imputables à des infections phagiques des levains lactiques continuent à survenir et entraînent des pertes économiques importantes.

D'autre part, l'absence de test spécifique approprié à une détection précoce, rapide, simple et fiable de contaminants bactériophagiques dans les levains et dans les matières premières explique et entretient les difficultés actuelles des fabrications de produits fermentés.

La présente invention s'est donné pour objet la mise au point d'un matériel et d'un procédé de détection utilisables industriellement dans le contrôle des matières premières et dans les suivis de fabrication, ainsi que la mise au point d'un produit et d'un procédé utilisables dans la lutte contre les bactériophages lactiques. Il s'agit, là, en effet, de voies nouvelles de progrès technique et économique susceptibles de satisfaire rapidement les besoins actuels des industriels.

Un test de diagnostic décrit dans le EP-A-0 143 107 utilise des antigènes bactériens déposés ou couplés sur les parois des puits de la plaque. Le phage est utilisé dans un premier temps, comme marqueur spécifique de cet antigène bactérien. Dans un deuxième temps, un ou deux anticorps antiphage sont utilisés pour révéler la fixation et pour identifier indirectement l'antigène bactérien.

Ce procédé utilise donc les phages pour détecter des antigènes bactériens, ces antigènes étant eux-mêmes révélés dans un deuxième temps par un ou deux anticorps greffés à une enzyme.

L'invention concerne un mélange d'anticorps spécifiques de bactériophages de bactéries lactiques des genres Streptocoque, Lactobacille, Pédiocoque et Leuconostoc, ainsi que ses applications à la détection et à la neutralisation desdits bactériophages.

Ainsi, selon l'invention, le phage n'est plus un outil de détection, mais constitue, par contre, l'antigène recherché grâce à l'anticorps monoclonal correspondant.

Il convient de souligner que ces anticorps monoclonaux sont spécifiques de tous les bactériophages de bactéries lactiques pouvant être rencontrés dans la nature. C'est là un des aspects nouveaux et originaux de la présente invention, étant entendu que jusqu'à présent, les publications faites dans ce domaine, concernent soit certaines souches de phages de Streptocoque lactique, soit certaines souches de phages de Lactobacille.

Seul un travail fondamental approfondi a permis de classer les différents groupes de phages rencontrés dans la nature, d'identifier des antigènes spécifiques de ces groupes, et de préparer les anticorps monoclonaux correspondants. Ceci constitue une différence et un progrès important par rapport aux publications antérieures.

En effet, Kim Y.K. et al, Sanop Misaengmul Hakhoechi, 1985, 13(3), 265-71 cité dans Chemical Abstracts, vol.103, N°25, 23 décembre 1985, pages 494-495 - résumé N° 21058-2, proposent une classification des bactériophages strictement limitée aux souches de Lactobacillus caséi S-1.

L'article de Bucholc et al dans Med. Dosw. Mikrobiol 27(4) 379-387, 1975, cité dans Biological Abstracts, vol 62, 15 août 1976, page 1973, résumé N° 19956 concerne la vaccination de bovins contre des phages obtenus à partir de Streptococcus lactis, Streptococcus diacetyllactis et Streptococcus cremoris ; on étudie les différentes modalités d'immunisation pour obtenir des anticorps actifs dans le lait de façon à lutter contre les infections phagiques durant la fabrication des fromages.

Compte-tenu de la date de cette publication et bien qu'aucune précision ne soit apportée sur la nature des groupes phagiques contenus dans les antigènes de phages de Streptocoque utilisé, on peut estimer que les phages utilisés comme antigènes dans cette étude ne représentaient pas l'ensemble des groupes.

Or, l'Inventeur, après un travail de recherche fondamentale, a isolé trois groupes de phages, biochimiquement et génétiquement différents, pour les Streptocoques mésophiles ; ces trois groupes de phages constituent trois groupes d'antigènes. En outre, dans le mélange de sérums, objet de la présente invention, on utilise aussi les phages préparés chez les Streptocoques thermophiles (Streptococcus thermophilus), ainsi que chez les Lactobacilles (Lactobacillus bulgaricus, Lactobacillus helveticus et Lactobacillus lactis).

Les immuno-globulines ainsi obtenues sont polyspécifiques et actives contre tous les phages de bactéries lactiques (Streptococcus lactis, cremoris, diacetyllactis, thermophilus, Lactobacillus lactis, bulgaricus, helveticus...). Cela n'était pas le cas des préparations d'anticorps faites en laboratoire jusqu'à ce jour. Ce mélange d'anticorps spécifiques et polyvalents permet, par ailleurs, soit le traitement des ferments lactiques dans une étape de sérothérapie in vitro lors de la fabrication de ces mêmes ferments lactiques, soit le traitement des cuves de fermentation fromagère par adjonction au moment de la fabrication du fromage. Le mode

4

d'utilisation de ces anticorps s'avère donc là aussi nouveau par rapport à l'acquis antérieur.

Le mélange d'anticorps anti-bactériophages lactiques selon l'invention est caractérisé en ce qu'il est obtenu par immunisation d'animaux à l'aide d'antigènes constitués par des sérotypes de bactériophages lactiques rencontrés dans la nature ou en laboratoire et actifs contre les bactéries lactiques mésophiles et thermophiles telles que les Streptocoques lactiques, les Lactobacilles, les Pédiocoques et les Leuconostocs.

Selon un mode de réalisation préféré de l'invention, les antigènes utilisés dans l'immunisation sont constitués par un mélange de l'ensemble des différents sérotypes des bactériophages actifs contre les bactéries lactiques telles que les Streptocoques lactiques, Lactobacilles, Pédiocoques, Leuconostocs.

Avantageusement, les anticorps sont polyspécifiques vis-à-vis des phages des bactéries lactiques, de nature polyclonale et obtenus par immunisation des ovidés, capridés, bovidés, et rongeurs.

Selon un autre mode de réalisation, les anticorps sont de nature monoclonale et obtenus à partir de muridés.

Les anticorps obtenus après immunisation, prélèvement et saignée d'animaux, sont ensuite fractionnés et titrés en vue d'obtenir une préparation à haut titre neutralisant vis-à-vis des bactériophages des bactéries lactiques.

L'invention concerne également l'utilisation du mélange d'anticorps ainsi obtenus à titre curatif et neutralisant dans les milieux biologiques tels que les laits ou les cultures bactériennes initialement contaminées par les bactériophages.

L'invention concerne enfin l'utilisation dudit mélange d'anticorps dans la détection immunologique des bactériophages présents dans un milieu contaminé (levain, lait, lacto-sérum, etc...).

La présente invention sera mieux comprise et ses avantages ressortiront bien des exemples qui l'illustrent, sans nullement la limiter.

Exemple 1 : <u>Préparation d'un mélange d'anticorps polyclonaux anti-bactériophages lactiques à usage curatif</u>

L'un des produits de l'invention est constitué par un mélange d'anticorps de bovins immunisés contre les bactériophages des bactéries lactiques (bactériophages spécifiques de Streptocoques lactiques, bactériophages spécifiques de Lactobacilles, bactériophages spécifiques de Pédiocoques, bactériophages spécifiques de Leuconostocs) obtenus et utilisés comme suit :

a) Préparation du mélange d'antigènes

Selon l'invention, on utilise pour obtenir des anticorps polyspécifiques, des antigènes d'immunisation constitués par un mélange de l'ensemble des différents sérotypes de bactériophages lactiques existant soit dans les collections officielles, soit dans la nature (levains lactiques, laits, lacto-sérums, etc...).

Ces différents sérotypes viraux constituent les antigènes et sont obtenus par multiplication sur des souches de bactéries lactiques productrices en phase exponentielle de multiplication infectées à raison de dix phages par bactérie.

A la fin du cycle lytique, le milieu de culture est centrifugé à température ambiante à 1 500 g pendant quinze minutes en vue de sédimenter les débris des corps bactériens lysés.

Le surnageant contenant la suspension virale est ensuite concentré dix fois par ultra-filtration par exemple sur membrane Millipore à seuil de coupure 100 000.

Le concentré viral ainsi obtenu est alors ultra-centrifugé sur gradient de saccharose.

Le précipité viral purifié est dialysé contre de l'eau distillée afin d'éliminer le saccharose et il constitue la préparation enrichie, purifiée d'antigènes titrant généralement $10^{11}$ particules virales par ml.

b) Préparation du mélange d'anticorps

Des bovins adultes, en bonne santé, indemnes de maladies infectieuses, vaccinés contre la fièvre aphteuse, de préférence de race homogène, sont utilisés pour l'immunisation.

Celle-ci est entreprise par injection par voie sous-cutanée de 10 ml du mélange d'antigènes viraux adjuvés à volume égal par un mélange d'hydroxyde d'alumine et de saponine.

Les bovins reçoivent des injections de rappel par voie sous cutanée les dixième, vingtième, trentième et quarantième jours suivants.

Ils sont ensuite abattus et leur sang est recueilli sur solution anti-coagulante puis centrifugé sur décanteuse en vue d'obtenir le plasma.

Celui-ci peut alors être fractionné par les techniques classiques chimiques en vue de l'obtention d'immuno-globulines purifiées (on peut, par exemple, utiliser un fractionnement par une solution de sulfate d'amonium saturé).

Les immuno-globulines dialysées et purifiées ainsi obtenues sont concentrées par ultra-filtration sur membrane à seuil de coupure de 20 000 puis elles sont filtrées et stabilisées en solution à 100 grammes par litre.

L'activité biologique de cette préparation est alors déterminée par neutralisation in vitro sur un mélange Streptocoques lactiques/bactériophages et sur un mélange lactobacilles/bactériophages.

Le titre inhibiteur de la préparation dans ces systèmes atteint généralement 1 000 unités neutralisantes par ml d'anticorps.

La préparation peut également être stabilisée par lyophilisation.

c) Utilisation

Ce mélange d'anticorps anti-bactériophages est utilisé comme additif neutralisant et curatif par adjonction dans les laits de vache destinés à la fabrication fromagère et contaminés par des bactériophages. Il doit, dans ce cas, être obligatoirement composé de protéines homologues à l'espèce qui a fourni le lait et doit répondre aux impératifs de non toxicité et d'activité des additifs alimentaires.

Ce mélange peut également être utilisé comme un additif curatif de fabrication lors de la préparation industrielle de levains lactiques contaminés par les bactériophages.

Exemple 2 : Préparation d'anticorps monoclonaux anti-bactériophages lactiques à usage diagnostic

a) La préparation d'anticorps monoclonaux fait appel à l'immunisation de souris, par exemple de la lignée Balb/c, immunisées à raison de 0,1 ml, par voie intrapéritonéale d'un mélange de suspensions de différents sérotypes de bactériophages lactiques obtenus dans les conditions de l'exemple 1 (a).

Les souris immunisées peuvent être sacrifiées 10 à 15 jours plus tard. Leur rate est prélevée, dissociée et leurs lymphocytes sont fusionnées avec une lignée de myélome de souris, puis mis en culture, en micro-plaques, sur milieu HAT sélectif.

Après plusieurs jours de culture, l'activité immunologique anti-phages des surnageants est contrôlée et les clones producteurs d'anticorps anti-phages sont ré-isolés puis multipliés par ascite induite chez la souris.

Le liquide d'ascite induite par les hybridomes sécrétants est alors récolté puis purifié par chromatographie d'affinité.

Les anticorps monoclonaux actifs contre les différents antigènes de bactériophages sont alors mélangés et utilisés pour la fabrication du test diagnostic.

b) Utilisation

Selon un exemple non limitatif, ces anticorps monoclonaux anti-bactériophages lactiques sont greffés dans des concentrations optimales par réaction chimique à la glutaraldéhyde sur des micro-billes de polyacrylamide métallisées (billes de polyacrylamide métallisées contenant des particules de fer par exemple).

Ces micro-billes à anticorps greffés sont utilisées pour fixer les antigènes bactériophagiques éventuellement présents dans un lait ou dans une culture de levains contaminés.

La réaction antigènes-anticorps est ensuite mise en évidence par une réaction immuno-enzymatique simple ou amplifiée à l'aide d'un anticorps anti-phages couplé à une enzyme capable de dégrader un substrat en fournissant une réaction colorée (réaction Elisa).

Les différentes étapes de la réaction Elisa peuvent être réalisées de manière simplifiée à l'usine grâce à un aimant qui accélère le contact et la séparation des billes de polyacrylamide métallisées et sensibilisées par les anticorps monoclonaux.

Ce test immunologique de type Elisa, fourni à titre d'exemple, peut aussi faire appel, dans le cadre de l'invention, à un mélange d'anticorps anti-bactériophages lactiques polyclonaux obtenus par exemple chez le lapin, le mouton ou le bovin, comme indiqué précédemment.

## - REVENDICATIONS -

1- Mélange d'anticorps anti-bactériophages lactiques, caractérisé en ce qu'il est obtenu par immunisation d'animaux à l'aide d'antigènes constitués par des sérotypes de bactériophages lactiques rencontrés dans la nature ou en laboratoire et actifs contre les bactéries lactiques mésophiles et thermophiles telles que les Streptocoques lactiques, les Lactobacilles, les Pédiocoques et les Leuconostocs.

2- Mélange selon la revendication 1, caractérisé en ce que les antigènes utilisés dans l'immunisation sont constitués par un mélange de l'ensemble des différents sérotypes des bactériophages actifs contre les bactéries lactiques telles que les Streptocoques lactiques, Lactobacilles, Pédiocoques, Leuconostocs.

3- Mélange selon la revendication 1 et la revendication 2, caractérisé en ce que les anticorps sont polyspécifiques vis-à-vis des phages des bactéries lactiques, de nature polyclonale et en ce qu'ils sont obtenus par immunisation des ovidés, capridés, bovidés, et rongeurs.

4- Mélange selon la revendication 1 et la revendication 2, caractérisé en ce que les anticorps sont de nature monoclonale et en ce qu'ils sont obtenus à partir de muridés.

5- Mélange selon l'une quelconque des revendications 1 à 4, caractérisé en ce que les anticorps obtenus après immunisation, prélèvement et saignée d'animaux, sont ensuite fractionnés et titrés en vue d'obtenir une préparation à haut titre neutralisant vis-à-vis des bactériophages des bactéries lactiques.

6- Application à titre curatif et neutralisant dans les milieux biologiques tels que les laits ou les cultures bactériennes initialement contaminées par les bactériophages du mélange selon l'une quelconque des revendications 1 à 5.

7- Application à la détection immunologique des bactériophages présents dans un milieu contaminé (levain, lait, lacto-sérum, etc...) du mélange selon l'une quelconque des revendications 1 à 5.

Office européen
des brevets

RAPPORT DE RECHERCHE EUROPEENNE

Numero de la demande

0228975

EP 86 42 0292

## DOCUMENTS CONSIDERES COMME PERTINENTS

| Catégorie | Citation du document avec indication. en cas de besoin. des parties pertinentes | Revendication concernee | CLASSEMENT DE LA DEMANDE (Int Cl 4) |
|---|---|---|---|
| D,X | BIOLOGICAL ABSTRACTS, vol. 62, 15 Août 1976, page 1973, résumé no. 19956, Philadelphia, Pa., US; B. BUCHOLC et al.: "Antibody activity of blood serum and whey of cows immunized with phages of group N streptococci", & MED. DOSW. MIKROBIOL. 27(4), 379-387, 1975 * En entier * | 1-3,5, 6 | C 07 K 15/00<br>C 12 P 21/00<br>A 61 K 39/42<br>G 01 N 33/02<br>G 01 N 33/04<br>G 01 N 33/569 //<br>C 12 N 15/00<br>(C 12 P 21/00<br>C 12 R 1:91 ) |
| D,X | EP-A-0 143 107 (J. LEMBKE) * En entier * | 1-5,7 | |
| D,X | CHEMICAL ABSTRACTS, vol. 103, no. 25, 23 décembre 1985, pages 494-495, résumé no 210585r, Columbus, Ohio, US; Y.K. KIM et al.: "Classifcation of bacteriophage of lactobacillus casei strain S-1", & SANOP MISAENGMUL HAKHOECHI 1985, 13(3), 265-71 * En entier * | 1-3,5, 6 | |

DOMAINES TECHNIQUES RECHERCHES (Int. Cl.4)

C 12 P
C 12 Q
A 61 K
G 01 N

--- -/-

Le présent rapport de recherche a été établi pour toutes les revendications

| Lieu de la recherche | Date d'achèvement de la recherche | Examinateur |
|---|---|---|
| LA HAYE | 04-03-1987 | GRIFFITH G. |

## DOCUMENTS CONSIDERES COMME PERTINENTS

**Page 2**

| Catégorie | Citation du document avec indication, en cas de besoin, des parties pertinentes | Revendication concernée | CLASSEMENT DE LA DEMANDE (Int. Cl.4) |
|---|---|---|---|
| A | BIOLOGICAL ABSTRACTS, vol. 81, no. 5, 1986, page AB-565, résumé no 44120, Philadelphia, Pa., US; S.A. HALPERIN et al.: "ELISA for identifcation and measurement of antibodies to group A streptococcal bacteriophage", & J. LAB. CLIN. MED., 106(5), 505-511, 1985 * En entier * | 1-3,5, 7 | |
| A | BIOLOGICAL ABSTRACTS, vol. 78, no. 4, 1984, page 2904, résumé no. 25566, Philadelphia, Pa., US; Yu. MANTSYGIN et al.: "The advantage of swine serum used for cultivation of some mammalian and human cell lines", & TSITOLOGIYA, 25(10), 1197-1201, 1983 * En entier * | 4 | **DOMAINES TECHNIQUES RECHERCHES (Int. Cl.4)** |
| A | BIOLOGICAL ABSTRACTS, vol. 81, no. 4, 1986, page AB-654, colonne 2, résumé no. 35126, Philadelphia, Pa., US; Yu. MANTSYGIN et al.: "Swine serum increases stability of hybridomas", & TSITOLOGIYA, 27(7), 822-827, 1985 * En entier * | 4 | |

Le présent rapport de recherche a été établi pour toutes les revendications

| Lieu de la recherche | Date d'achevement de la recherche | Examinateur |
|---|---|---|
| LA HAYE | 04-03-1987 | GRIFFITH G. |